# EUROPEAN PATENT APPLICATION

(11) **EP 1 801 225 A1**
(43) Date of publication of application: **27.06.2007**
(21) Application number: 05774551.5
(22) Date of filing: 18.08.2005
(51) Int. Cl.: C12P 7/64, C12N 1/14, C11C 3/00

(54) **PROCESS FOR PRODUCING TRIGLYCERIDE CONTAINING THREE RESIDUES OF ONE HIGHLY UNSATURATED FATTY ACID AND USE THEREOF**

(30) Priority: 24.08.2004 JP 2004244218
(71) Applicant: SUNTORY LIMITED, Osaka-shi, Osaka 530-8203 (JP)
(72) Inventor: KAWASHIMA, Hiroshi, Takatsuki-shi, Osaka 5691121 (JP); SAKURADANI, Eiji, GokashoUji-shi, Kyoto 6110011 (JP); OGAWA, Jun, Kyoto-shi, Kyoto 6038051 (JP); SHIMIZU, Sakayu, Kyoto-shi, Kyoto 6168212 (JP)
(74) Representative: Vossius, Volker
(86) International application number: PCT/JP2005/015380
(87) International publication number: WO 2006/022310

(57) **Abstract**

The invention provides a process, for producing fats or oils in which the rate of triglycerides constituted by the same polyunsaturated fatty acids linking to the three hydroxyl groups of glycerol is 20% or more by weight for the total triglycerides, which comprises culturing a microorganism capable of producing said fats or oils and if required recovering said fats or oils; fats or oils produced by this process; and a process for producing said fats or oils.

## Description

### Technical Field

The present invention relates to a process for producing triglycerides constituted by three polyunsaturated fatty acid residues of one type and the utilization thereof. In particular, it relates to a process for producing the above triglycerides efficiently and stably, the triglycerides thus produced by said process, and a representative utilization of them.

### Background Art

The polyunsaturated fatty acids (PUFAs) mentioned herein mean fatty acids which contain 18 or more carbons and have 2 or more of double bonds. Since PUFAs have a variety of unique physiological activities, they have been used as additives to many kinds of food and animal feed to enhance their functionality. The major ones include linoleic acid (LA), α-linolenic acid (ALA), γ-linolenic acid (GLA), dihomo-γ-linolenic acid (DGLA), mead acid (MA), arachidonic acid (AA), eicosapentaenoic acid (EPA), docosahexaenoic acid (DHA), and the like. In utilization, they may be used mainly in a form of triglycerides in which a PUFA is contained as a constituent in the acyl residue in many cases, though they may be used in some cases as free fatty acids or phospholipids.

In general, since the chemical synthesis of a PUFA itself is difficult and is extremely costly, a practical source of supply is limited to extraction from biological sources. In many cases, PUFA is extracted generally from oil plants, seafood, microorganisms, fine algae, and the like. These organisms usually contain a variety of PUFAs, and biological sources comprising a single PUFA are not generally known.

In utilizing PUFAs in a form of triglycerides, it has recently been known that the functionality of PUFA can further be enhanced by using a PUFA as the so-called "structured lipid" in which the binding site and binding number of a PUFA are specified. In producing the structured lipids, in many cases, since PUFAs are mainly used as foods, they are produced by a conversion reaction of an acyl group with an enzyme such as lipase, though a site-specific chemical procedure is used in some cases (Japanese Patent JP-A 2003-4831, P01-0044). In this case, in general, the linkage of the fatty acid linking to the starting triglyceride at a specific site is not cleaved, but the only fatty acid(s) linking to the other site(s) is (are) mainly removed by hydrolysis or exchange of an acyl group(s), though it depends on a design of a reaction system. The reaction sequence 1 indicates an example of generation of triglycerides containing caprylic acid at the 1 and 3 positions and free fatty acids from a PUFA-containing triglyceride mixture and caprylic acid by reaction of lipase (wherein A to I: fatty acid; 8: caprylic acid).

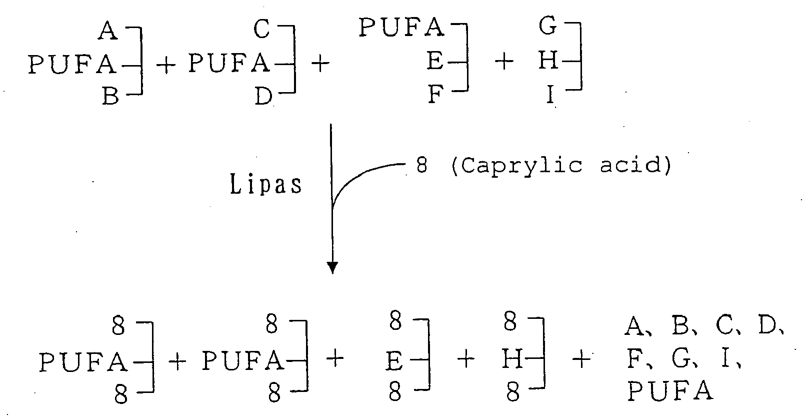
A          C          PUFA     G
PUFA  +  PUFA  +       E  +  H
B          D            F     I


Lipas          8 (Caprylic acid)

8          8           8     8         A、B、C、D、
PUFA  +  PUFA  +  E  +  H  +  F、G、I、
8          8           8     8         PUFA

In this case, the content of the objective PUFA in the product structured lipid depends on the content of the objective PUFA contained in the uncleaved fatty acid residue (that at the 2 position in this case) in the starting triglyceride. The sort and rate of the fatty acid included in this free fatty acid depend on the sort and rate of the fatty acid contained in the cleaved fatty acid residue (those at the 1 and 3 positions in this case) in the starting triglyceride, though high utility value is expected in by-product free fatty acids in many cases.

When structured lipids containing a specific PUFA at a specific site are produced starting from triglycerides or their equivalents containing the PUFA by a conversion reaction of an acyl group with lipase intending to utilize by-product fatty acids incidentally, use of a mixture of a variety of PUFAs as starting materials causes the following problems. (1) Fatty acids other than the aimed PUFA would be mixed with the acyl group which is expected to remain linked to the glycerol skeleton. (2) The by-product fatty acids released from the glycerol skeleton form a mixture of various kinds of fatty acids.

In order to solve the above-mentioned problems (1) and (2) at the same time, triglycerides in which all three constituting fatty acid residues comprise one type of the objective PUFA may be used as starting materials. Thus, it is possible to obtain highly pure and highly valuable products comprising only one type of the objective PUFAs, that is, in the acyl group which is expected remaining linked to the glycerol skeleton and in the by-product fatty acids released from the glycerol skeleton.

When a human or animal is intended to ingest lipids containing PUFAs expecting a specific physiological effect of PUFAs, they are ingested in many cases as triglycerides in view of their physicochemical properties such as stability and low irritation to the body, but there is sometimes a problem regarding calories because they are lipids. In such a case, if a triglyceride in which all of the three constitutive fatty acid residues comprise only one type of the objective PUFA is ingested, since no other fatty acid is needed, it would be expected as another effect that the calorie taken from an equal amount of the objective PUFA is the lowest.

Thus, triglycerides in which all of the three constitutive fatty acid residues comprise only one type of the objective PUFA are materials having very high added value.

When the molecular species of triglyceride comprising one type of the objective PUFA, even if it is small, is contained in a mixture of triglycerides containing the objective PUFA, it can be purified in some cases by separation using high performance liquid chromatography. Alternatively, it is also possible theoretically to obtain a triglyceride in which all of the three constitutive fatty acid residues comprise one type of the objective PUFA, by converting a lipid containing the objective PUFA into a free fatty acid or fatty acid alcohol ester, then purifying by separation using high performance liquid chromatography to give one type of the objective PUFA, and further binding the latter to glycerol by chemical synthesis or enzymatic conversion.

### Disclosure of Invention

In the above-mentioned prior art, however, there is a problem that it is difficult to efficiently and stably produce a triglyceride comprising three PUFA residues of one type.

Further, in general and as mentioned above, as the chemical synthesis of PUFA per se is hard and has a very high cost, a practical source of supply is limited to extraction from biological sources. A major source of supply includes oil plants for linoleic acid and α-linolenic acid, microorganisms for arachidonic acid and dihomo-γ-linolenic acid, and seafood or fine algae for EPA and DHA. In many cases, the PUFAs contained in these sources exist in forms of triglycerides.

The triglycerides, however, contain a wide variety of PUFAs of which the binding position to glycerol is distributed to all of 1, 2 and 3 positions of glycerol skeleton in many cases. Thus, in view of the triglyceride molecular species which distinguishes the sort of the three fatty acid residues on triglyceride, there are a large number of molecular species, and therefore almost no molecular species of triglycerides in which all of the three constitutive fatty acid residues comprise one type of the objective PUFA is contained, or if contained their content would be very low, indicating that it is difficult to separate and purify a molecular species of triglycerides in which all of the three constitutive fatty acid residues comprise one type of the objective PUFA from the above-mentioned sources of supply. Moreover, it is necessary to use analytical equipment, such as one for high performance liquid chromatography, in order to separate and purify a specific molecular species of triglycerides from triglyceride mixtures, indicating that mass production in an industrial scale is very difficult.

In addition, it is possible to obtain triglycerides in which all of the three constitutive fatty acid residues are one type of the objective PUFA, by separating and purifying one type of the objective PUFA in a form of free fatty acids or fatty acid alcohol esters and then binding it to glycerol by chemical synthesis or enzymatic conversion. In this case, however, the objective PUFA per se can be obtained only from the above-mentioned sources of supply and, further, many more steps are required, for example, extraction of fats or oils, conversion into free fatty acids or fatty acid alcohol esters, separation and purification of the objective PUFAs, binding to glycerol by chemical synthesis or enzymatic conversion, and separation and purification of triglycerides constituted by one type of the objective PUFA. This process, accordingly, was not practical because there was a big problem in cost and yield.

The present invention was made in consideration of the above problems and the purpose of the invention is to provide a technique permitting efficient and stable supply of triglycerides constituted by 3 PUFA residues of one type and a typical technique for utilizing it.

The present inventors worked assiduously in the light of the above-mentioned problems, and found that mutation treatment of a lipid-producing microorganism which produces a triglyceride containing the objective PUFA afforded a mutant containing a high concentration of triglyceride in which all of the three constitutive fatty acid residues are one type of the objective PUFA, and that a triglyceride in which all of the three fatty acid residues constituting triglyceride are one type of the objective PUFA can readily be extracted from the cells of said mutant. Thus, the present invention was completed.

According to the invention, a process for producing a triglyceride constituted by 3 PUFA residues of one type comprises culturing a lipid-producing microorganism capable of producing triglycerides constituted by 3 PUFA residues of one type and extracting from the resulting cells the triglycerides constituted by 3 PUFA residues of one type, wherein the process is characterized by culturing a lipid-producing microorganism capable of producing triglycerides constituted by 3 PUFA residues of one type.

Accordingly, the invention provides a process for producing fats or oils in which the rate of triglycerides constituted by the same polyunsaturated fatty acids linking to the three hydroxyl groups of glycerol is 20% or more by weight for the total triglycerides, which comprises culturing a microorganism capable of producing said fats or oils and if required recovering said fats or oils.

In the above-mentioned process, the rate of triglycerides constituted by the same polyunsaturated fatty acids linking to the three hydroxyl groups of glycerol is preferably 30% or more by weight for the total triglycerides.

More preferably, in the above-mentioned process, the amount of triglycerides constituted by the same polyunsaturated fatty acids linking to the three hydroxyl groups of glycerol is 40% or more by weight for the total triglycerides, and even more preferably, the amount of triglycerides constituted by the same polyunsaturated fatty acids linking to the three hydroxyl groups of glycerol is 50% or more by weight for the total triglycerides.

In the above-mentioned process, the above-mentioned same polyunsaturated fatty acid is, for example, arachidonic acid.

The microorganism used in the above-mentioned process is preferably one belonging to a genus Mortierella, and more preferably one of a subgenus Mortierella of a genus Mortierella. Preferably, the above-mentioned microorganism is one of a species Mortierella alpina. Usually, the above-mentioned microorganism is a mutant derived from a microorganism capable of producing fats or oils containing polyunsaturated fatty acids.

The invention also relates to fats or oils produced by the above-mentioned process.

Furthermore, the invention provides a microorganism of a genus Mortierella which is able to produce fats or oils in which the rate of triglycerides constituted by the same polyunsaturated fatty acids linking to the three hydroxyl groups of glycerol is 20% or more by weight for the total triglycerides; preferably a microorganism of a genus Mortierella which is able to produce fats or oils in which the rate of triglycerides constituted by the same polyunsaturated fatty acids linking to the three hydroxyl groups of glycerol is 30% or more by weight for the total triglycerides. More preferably, the microorganism is, for example, Mortierella alpina. The above-mentioned microorganism is preferably a mutant and, for example, an artificial mutant.

The invention also provides fats or oils in which the rate of triglycerides constituted by the same polyunsaturated fatty acids linking to the three hydroxyl groups of glycerol is 20% or more by weight for the total triglycerides; preferably, fats or oils in which the rate of triglycerides constituted by the same polyunsaturated fatty acids linking to the three hydroxyl groups of glycerol is 30% or more by weight for the total triglycerides; more preferably, fats or oils in which the rate of triglycerides constituted by the same polyunsaturated fatty acids linking to the three hydroxyl groups of glycerol is 40% or more by weight for the total triglycerides; and even more preferably, fats or oils in which the rate of triglycerides constituted by the same polyunsaturated fatty acids linking to the three hydroxyl groups of glycerol is 50% or more by weight for the total triglycerides.

The invention further provides the cultured cells of microorganism containing fats or oils in which the rate of triglycerides constituted by the same polyunsaturated fatty acids linking to the three hydroxyl groups of glycerol is 20% or more by weight for the total triglycerides. Preferably, the rate of triglycerides constituted by the same polyunsaturated fatty acids linking to the three hydroxyl groups of glycerol is 30% or more by weight for the total triglycerides; more preferably, the rate of triglycerides constituted by the same polyunsaturated fatty acids linking to the three hydroxyl groups of glycerol is 40% or more by weight for the total triglycerides; for example, the rate of triglycerides constituted by the same polyunsaturated fatty acids linking to the three hydroxyl groups of glycerol is 50% or more by weight for the total triglycerides.

### Effect of the Invention

In the invention, the efficient and stable production of easily available triglycerides containing the 3 PUFA residues of one type will give the following satisfactory results.

When the structured lipids containing a specific PUFA at a specific site are produced by an exchange reaction of the acyl group using lipase, where the by-product fatty acids are also intended to be used, the use of the starting triglyceride containing the 3 PUFA residues of one type of the invention affords highly pure products comprising only one type of the objective PUFAs, in the acyl group which is expected remaining linked to the glycerol skeleton and in the by-product fatty acids released from the glycerol skeleton. Thus, the added values of the main products and by-products are greatly increased.

On ingestion of a PUFA-containing lipid expecting a specific physiological effect of PUFA by a human or animal, when a triglyceride in which all of the three constitutive fatty acid residues are one type of the objective PUFA is taken, an effect is expected that the calorie to be taken to ingest an equal amount of the objective PUFA is kept at the least level as no other fatty acid is necessary to be taken.

### Best Mode for Carrying Out the Invention

One mode for carrying out the invention will be explained as follows, but is not intended to limit the scope of the invention.

According to the invention, a process for producing a triglyceride constituted by 3 PUFA residues of one type comprises culturing a lipid-producing microorganism capable of producing triglycerides constituted by 3 PUFA residues of one type and extracting from the resulting cells the triglycerides constituted by 3 PUFA residues of one type, wherein the process is characterized by culturing a lipid-producing microorganism capable of producing triglycerides constituted by 3 PUFA residues of one type.

The above-mentioned lipid-producing microorganism used in the invention preferably includes but is not limited to at least one species selected from the genera Mortierella, Conidiobolus, Pythium, Phytophthora, Penicillium, Cladosporium, Mucor, Fusarium, Aspergillus, Rhodotorula, Entomophthora, Echinosporangium, and Saprolegnia.

Among these microorganisms, when a genus Mortierella is used as a productive microorganism in production of the above lipids, it is preferably a species of a subgenus Mortierella, more preferably Mortierella alpina. The microorganism belonging to the genus Mortierella, subgenus Mortierella, includes, for example, Mortierella elongata, Mortierella exigua, Mortierella hygrophila, Mortierella alpina, and the like.

Specifically, Mortierella elongata (IFO8570), Mortierella exigua (IFO8571), Mortierella hygrophila (IFO5941), Mortierella alpina (IFO8568, ATCC16266, ATCC32221, ATCC42430, CBS219.35, CBS224.37, CBS250.53, CBS343.66, CBS527.72, CBS529.72, CBS608.70, CBS754.68) and so on are included.

In addition to the subgenus Mortierella, as for the above-mentioned lipid-producing microorganisms, the following strains can be used: Echinosporangium transversalis (ATCC 16960), Conidiobolus heterosporus (CBS138.57), Saprolegnia lapponica (CBS284.38) and the like.

These microorganisms are available from Institute for Fermentation, Osaka (IFO), American Type Culture Collection (ATCC), and Centrralbureau voor Schimmelcultures (CBS), without any restriction. In addition, another strain Mortierella alpina SAM2268 (FERM P-17762) which was isolated from soil by a research group of the present inventors can be used. The invention, however, is not limited to these microorganisms. In the invention, though the microorganisms belonging to these type cultures or those isolated from the natural sources can be used as such, a spontaneous mutant derived from an original strain by single or plural multiplication and/or isolation can be used as a strain different from the original strain in its property.

Further, it is possible to select a lipid-producing strain of which the productivity of triglycerides constituted by 3 PUFA residues of one type has been enhanced, by mutating one of the above lipid-producing strain followed by selection. A procedure for mutation can be effected without any particular limitation as far as it is applicable to the above-mentioned lipid-producing strains, and includes irradiation with radiation (X-ray, gamma ray, neutron beam) or ultraviolet ray, and treatment under heating at high temperatures. Alternatively, said microorganism may be suspended in an appropriate buffer, to which a mutagen is added and incubated for a certain period of time, then diluted properly, and inoculated on an agar plate to give a colony of the mutant. These general procedures for mutation can be applied.

The mutagen can include alkylating agents such as nitrogen mustard, methyl methanesulfonate or N-methyl-N'-nitro-N-nitrosoguanidine (NTG); base analogues such as 5-bromouracil; antibiotics such as mitomycin C; inhibitors for base synthesis such as 6-mercaptopurine; pigments such as proflavin; certain carcinogens such as 4-nitroquinoline-N-oxide; and compounds such as manganese chloride and formaldehyde. The microorganisms used may be in a form of growing cells (hyphae) or spores.

Selection of a lipid-producing strain of which the productivity of triglyceride constituted by 3 PUFA residues of one type has been enhanced from the mutated lipid-producing strain, may be carried out without any particular limitation, preferably by analyzing the triglycerides produced by the mutated lipid-producing strain by high performance liquid chromatography.

As shown in Example 1, approximately 3,000 strains which have been mutated were applied to selection as described above; among them three strains produced fats or oils containing triglycerides in a higher rate than others, which comprises only the same PUFA residue. This indicates that one objective strain could be obtained from 1,000 strains of the mutated microorganisms on the average. Therefore, the frequency of generation of the mutants in the invention is very high in comparison with other usual random selection by mutation and selection. A mutant which has the same character as the above-mentioned three mutants practically obtained in the invention can readily be generated by repeatedly carrying out the method as described in Example 1 of the invention.

A practical method for culturing a lipid-producing strain of which the productivity of triglyceride comprising 3 PUFA residues of one type has been enhanced is not limited particularly as far as the strain can be grown thereon to produce triglycerides, and it may be cultured in a conventional way according to the species of the lipid-producing strain. In general, the hypha, spores or a pre-cultured broth of a lipid-producing strain to be cultured is inoculated and incubated on a liquid culture medium or solid medium. When a large quantity of microorganism cells are required, liquid culture is preferred in many cases. There is no particular limitation in apparatus for culture. In a small scale culture, shaking culture with various test tubes or flasks containing a liquid medium or standing culture on an inoculated agar plate may be employed. In a large scale culture, various fermentation tanks or jar fermenters may be employed.

There is no particular limitation in the sort of culture media used in culture; the medium may be prepared by choosing properly conventional constituents according to the species of lipid-producing microorganisms. Alternatively, a medium in which the components are known or commercially available medium may be used as it is.

As for the liquid medium, the carbon source is not limited particularly, and conventional sugars can be used preferably. Specifically, for example, glucose, fructose, xylose, saccharose, maltose, soluble starch, molasses, glycerol, mannitol, and the like are included. These carbon sources may be used alone or in combination of two or more sorts.

The nitrogen sources are not limited particularly, and conventional ones may be used preferably. Specifically, for example, natural nitrogen sources such as peptone, yeast extract, malt extract, meat extract, casamino acid, corn steep liquor, soybean protein, defatted soybean, cotton seed sediment, and so on; organic nitrogen sources such as urea; and inorganic nitrogen sources such as sodium nitrate, ammonium nitrate, ammonium sulfate, and so on are included. In the invention, among these sources, though it depends on the sort of strain cultured, natural nitrogen source obtained from soybean, specifically soybean, defatted soybean, soybean flake, edible soybean proteins, bean-curd refuse, soymilk, soybean flour, etc. may preferably be used. Among them, thermally denatured defatted soybean, more preferably defatted soybean denatured by thermal treatment at about 70-90°C followed by removal of ethanol-soluble constituent, can be used. These nitrogen sources may be used alone or in combination of tow or more sorts.

There is no particular limitation in using other constituents than the above-mentioned carbon sources and nitrogen sources. If necessary, a known minor nutrient source may properly be added. The minor nutrient source includes, for example, inorganic acid ions such as phosphate ions; alkali metal or alkaline earth metal ions such as calcium ions, sodium ions, magnesium ions, calcium ions, and so on; metal ions of VIIB to VIII groups such as iron, nickel, cobalt, manganese, and so on; metal ions of IB to IIB groups such as copper, zinc, and so on; various vitamins, and so on.

The content (rate of addition) of the above-mentioned respective constituents in the liquid medium is not limited particularly, and may be within the known range as far as it does not inhibit the growth of lipid-producing strain. In practice, the total amount of the carbon sources to be added is generally within the range of 0.1 to 40% by weight, and more preferably within the range of 1 to 25% by weight. The total amount of the nitrogen sources to be added is within the range of 0.01 to 10% by weight, and more preferably within the range of 0.1 to 10% by weight. Further, in medium-feeding, the initial amount of the carbon source to be added is preferably within the range of 1 to 5% by weight, and the initial amount of the nitrogen source to be added is within the range of 0.1 to 6% by weight. The constituent of the medium to be fed during incubation may be both of the carbon source and nitrogen source, but more preferably the carbon source alone may be fed.

In the production process of the invention, it is appropriate to add a precursor of unsaturated fatty acid to the culture medium in order to increase the yield of triglyceride comprising 3 PUFA residues of one type. The precursor of unsaturated fatty acid specifically includes but is not limited to, for example, hydrocarbon such as hexadecane, octadecane, and so on; fatty acid or its salt such as oleic acid, linoleic acid, and so on; fatty acid ester such as ethyl ester, glycerin fatty acid ester, sorbitan fatty acid ester, and so on; and fats or oils such as olive oil, soybean oil, rapeseed oil, cotton seed oil, coconut oil, and so on. These precursors may be used alone or in combination of two or more sorts.

There is no particular limitation in the amount of the above-mentioned precursor of unsaturated fatty acid to be added. In general, however, the precursor may be added in the range of 0.001-10%, preferably in the range of 0.5-10%, for the total amount of the culture medium. The precursor may be used as the sole carbon source for the lipid-producing microorganism.

The condition for culture is not limited particularly, and may properly be determined according to the species of strain to be cultured. For example, the culture temperature may generally be in the range of 5-40°C, preferably in the range of 20-30°C. Alternatively, the culture may preliminarily be carried out in the range of 20-30°C to grow the microorganism, and then continued at 5-20°C. Such temperature control, that is, initial culture at a relatively high temperature and subsequent culture conducted at a lower temperature than the initial temperature, leads to increase of the rate of polyunsaturated fatty acid (PUFA) contained in the produced unsaturated fatty acids.

The pH in the culture medium is not particularly limited, and in general the culture may be conducted at pH 4-10, more preferably at pH 5-9. The term of the culturing is not limited particularly, and the culture is usually continued for a period of 2-30 days, preferably for a period of 5-20 days, and more preferably for a period of 5-15 days. External treatment given to the medium during culture is not limited particularly, and a known procedure for culture may properly be selected from aeration and agitation, shaking culture, standing culture, and the like.

When the solid culture is employed, wheat bran, rice hulls or rice bran to which 50-100% by weight of water for the total weight of solid material has been added is used, on which the culture is carried out at 5-40°C, preferably at a temperature as mentioned above, for a period of 3-14 days. If necessary, a nitrogen source, inorganic salts, and minor nutrient sources may be added.

In the invention, in order to enhance the yield of the triglyceride comprising 3 PUFA residues of one type, it is possible to add as a substrate a hydrocarbon such as tetradecane, hexadecane, and so on; fatty acid such as tetradecanoic acid, hexadecanoic acid, and so on, or its salt (e.g., sodium salt, potassium salt, etc.) and ester; or fats or oils containing said fatty acids as constitutive ingredients (e.g., coconut oil, palm kernal oil) and the like.

The condition for culture is not limited particularly, and may properly be determined according to the species of strain to be cultured. External treatment given to the medium during culture is not limited particularly, and a known procedure for culture may properly be selected from aeration and agitation, shaking culture, standing culture, and the like. Solid culture may also be employed.

The PUFAs which constitute triglycerides comprising 3 PUFA residues of one type concerning the invention may be fatty acids of 18 or more carbons having 2 or more double bonds. Specifically, for example, eicosadienoic acid; eicosatrienoic acid such as dihomo-γ-linolenic acid, mead acid; eiosatetraenoic acid such as arachidonic acid (AA); eicosapentaenoic acid; docosadienoic acid; docosatrienoic acid; docosatetraenoic acid; docosapentaenoic acid; docosahexaenoic acid (DHA); tetracosadienoic acid; tetracosatrienoic acid; tetracosatetraenoic acid; tetracosapentaenoic acid; tetracosahexaenoic acid; and the like, are included. Among the above-mentioned PUFAs, arachidonic acid (AA) is more preferably used.

In the above-mentioned PUFAs, at least one of the carbon-carbon double bonds (-C=C-) contained in the structure may be a conjugated double bond. This conjugated double bond may be conjugated with a carbonyl group (C=O) or with an adjacent carbon-carbon double bond.

The rate of the objective triglyceride constituted by 3 PUFA residues of one type to the total triglyceride produced by the above-mentioned lipid-producing microorganism is, but not limited to, preferably 30% or more by weight, more preferably 33% or more by weight, and even more preferably 45% or more by weight. When the triglyceride constituted by 3 PUFA residues of one type is tri-arachidonoyl glycerol, the rate of tri-arachidonoyl glycerol to the total triglycerides is, but is not limited to, preferably 30% or more by weight, more preferably 33% or more by weight, and even more preferably 45% or more by weight.

In the invention, the cells recovered in the culture step for the above-mentioned lipid-producing microorganism are subjected to a extraction step for fats or oils. In the extraction step for fats or oils, the collected cells may be used directly, i.e., as live cells, or after sterilization treatment. Alternatively, the culture broth may be treated directly without recovering the cells. The collected cells may be processed in an optional form before use. There is no particular limitation in a method for collection of the cells. When the cells are recovered in a small quantity, they may be collected by centrifugation using a conventional centrifuge. When the cells can be obtained in a large quantity, they may be collected by continuous centrifugation, preferably in combination with filtration through a membrane.

The collected cells may be used directly in a wet state or as dry cells after drying wet cells. In the invention, it is particularly preferred to use dry cells. By this, fats or oils can be extracted efficiently. Method for drying of wet cells includes but is not limited particularly to known methods for drying such as air-dry, thermal treatment, treatment under reduced pressure, lyophilization, and the like.

In the above extraction step for lipids and oils, there is no particular limitation in a procedure for extraction of fats or oils from the cells, and thus conventional extraction methods can be applied. Specifically, squeeze extraction under pressure, rendering extraction using hot water or steam, extraction with various extraction solvents, supercritical carbon dioxide, and the like are included.

In the squeeze extraction under pressure, the oil portion in the cells is squeezed by adding pressure to the raw material. Such a procedure specifically includes but is not limited to, for example, methods using an instrument such as a hydraulic press of batch type or an expeller of continuous type.

In the above rendering extraction, any method of dry-type and wet-type can be used, and specifically includes, but is not limited to, a drying method with direct flame, steam rendering with an autoclave (wet method), and the like.

The drying method is specifically explained as follows: for example, the cells are heated with direct flame or jacket steam to make fats elute. The steam rendering is specifically explained as follows: the cells are heated with stirring by bubbling hot steam to give oil portion in a form of emulsion together with water, proteins, and so on. This is applied to a centrifuge to remove waste water, and if required filtered to give raw oil. The condition of steam rendering includes but is not limited to, for example, elution with hot steam under 3-9 kg/cm² for a period of 4-6 hours.

In the above-mentioned extraction with extraction solvents, there is no particular limitation in the solvent used. In general, at least one of extraction solvents selected from aliphatic organic solvents and water, or supercritical carbon dioxide gas may be used. Among the above extraction solvents, the aliphatic organic solvent specifically includes, for example, saturated hydrocarbons such as hexane, petroleum ether (organic solvents containing pentane and hexane as major constituents), and so on; ketones such as acetone, and so on; alcohols such as methanol, ethanol, propanol, butanol, and so on; esters such as ethyl acetate, and so on; halogenated hydrocarbon such as chloroform, dichloromethane, and so on; cyanohydrocarbons such as acetonitrile, and so on; ethers such as diethyl ether; and so on.

Among the above extraction solvents, water may be used as an aqueous solution in which a known solute is dissolved. These extraction solvents may be used alone or in combination of two or more sorts. In order to efficiently extract fats or oils such as triglycerides, it is preferable to use a saturated hydrocarbon such as hexane or petroleum ether among the above extraction solvents, and hexane is more preferred.

Extraction with the above-described extraction solvents, may be achieved by batch-type or consecutive methods. The condition of extraction with an extraction solvent is not limited particularly. The extraction may be carried out at a proper temperature, with a proper amount of the solvent, for a proper period of time, according to the sort of fats or oils such as triglycerides to be extracted or the amount (volume and weight) of the microorganisms. In carrying out the extraction, the cells are dispersed in an extraction solvent, preferably with gentle stirring. Thus, efficient extraction can be achieved.

The fats or oils extracted in the above step for extraction can be used directly or, if required, further purified to increase the content of triglycerides. Purification can be achieved by a conventional purification method for fats or oils, which methods include but are not limited to removal of gum, deacidification, decolorization, deodorizaiton, filtration, wintering, molecular distillation, steam distillation, and the like. These methods may be used alone or in combination of two or more.

Thus, triglycerides, in which the content of the objective triglyceride constituted by 3 PUFA residues of one type is high, can be produced, and it is also possible to further increase the content of the objective triglyceride constituted by 3 PUFA residues of one type. Such a method includes but is not limited to column chromatography, urea inclusion, molecular distillation, and the like.

The invention can be utilized without any limitation. An example of the utilization is to use as a substrate (raw material) in enzyme reactions the objective triglyceride constituted by 3 PUFA residues of one type in the invention.

Another substrate to be used includes but is not limited to triglycerides, diglycerides, monoglycerides, free fatty acids, fatty acid alcohols esters, and the like. Preferably, animal or plant fats or oils, microbial fats or oils, fine algae fats or oils, middle chain fatty acid triglycerides, fatty acids of 2 to 24 carbons, and fatty acid alcohol esters of 2 to 24 carbons are used. More preferably, tricaproyl glycerol, caprylic acid, caprylic acid ester, and the like may be used, but the substrate is not limited thereto.

There is no particular limitation in the enzymatic reaction, and any type of reactions can be employed. Representatively, lipase reaction, phospholipase reaction, esterase reaction, and the like are included. The product obtained by the reaction includes functional fats or oils and free fatty acids; the functional fats or oils typically include but are not limited to structured lipids in which polyunsaturated fatty acids bind only at the 2 position, structured lipids in which polyunsaturated fatty acids bind only at the 1 and 3 position3, and structured lipids comprising polyunsaturated fatty acids and middle chain fatty acids. Preferred is a structured lipid in which caprylic acid binds at 1 and 3 positions and PUFA at 2 position. More preferably, a structured lipid in which caprylic acid binds at 1 and 3 positions and arachidonic acid or eicosatrienoic acid or eicosapentaenoic acid or docosahexaenoic acid at 2 position is exemplified. The free fatty acids obtained by the reaction are highly pure polyunsaturated fatty acids derived from the triglycerides constituted by 3 PUFA residues of one type concerning the invention.

The triglycerides constituted by 3 PUFA residues of one type concerning the invention can be used as supplemental nutritional compositions. There is no particular limitation in target organisms for the nutritional compositions, which can be applied to any kind of organism. Representative organisms include human, and additionally domestic animals and experimental animals. The nutritional compositions to be ingested may be in any forms, and most preferably the compositions are orally taken. Accordingly, foods containing the above-mentioned triglycerides constituted by 3 PUFA residues of one type are also included.

In the triglycerides constituted by 3 PUFA residues of one type concerning the invention, a variety of additives may be added to enhance their functionality. Specifically, such additives include but are not limited to, for example, sterols, sterol esters, glycolipids, sphingolipids, waxes, pigments, carotenoids, tocopherols, vitamin E, tocotrienol, sesamin, sesaminol, sesamol, astaxanthin, astaxanthin esters, sterols, carotenes, and the like. As the lipid compositions concerning the invention can be utilized as nutritional compositions, all kinds of additives which can be added to foods can be employed.

As the foods concerning the invention may contain the triglycerides constituted by 3 PUFA residues of one type concerning the invention, the kind of foods is not limited particularly. Specifically, such foods include but are not limited to bread, Japanese and western cakes (including frozen desserts), household dishes, dairy products, cereal foods, soybean curd and abura-age (fried thin slices of pressed tofu), noodles, packed lunch, seasonings, agricultural processed product such as wheat flour or edible meat, long-term preserved foods (canned food, frozen food, boil-in-the-bag food, etc.), beverage, milk-based drinks, soymilk, soup such as thick soup, and other general foods. There is no particular limitation in addition of the above triglycerides constituted by 3 PUFA residues of one type, and a conventional suitable way can be employed according to the kind of the general foods.

In addition, the foods concerning the invention include functional food used in a specific purpose different from the general foods, such as health food or nutritional food. A variety of nutritional supplemental foods such as supplement, special health food, and the like are specifically exemplified. The triglycerides constituted by 3 PUFA residues of one type concerning the invention, when used as supplement, may be processed into a proper shape, which is used directly. The processed shape is not limited particularly. Specifically, the lipid compositions (or foods) concerning the invention may be in forms of liquid or powder, or may be in forms of capsules or tablets or tablet-like preparations. Conventional techniques used in dissolution or pulverization of usual fat-types of foods all can be applied.

The invention can be applied to the field of medicines in addition to the field of foods. The triglycerides constituted by 3 PUFA residues of one type concerning the invention can be utilized as medicines. In utilization as medicines, they are not limited to specific examples, and a known technique may be employed according to the purpose.

### Example

The invention will be explained more specifically by the following examples which are not intended as limitations thereof.

### Example 1

### Generation of a triarachidonoyl glycerol high-production strain by mutation of Mortierella alpina SAM2268

Mortierella alpina SAM2268 (FERM P-17762) was inoculated in a big slant bottle containing 300mL of Czapek-agar medium (0.2% NaNO_{3,} 0.1% K₂HPO_{4,} 0.05% MgSO₄·7H₂O, 0.05% KCl, 0.01%.FeSO₉·7H₂O, 3% sucrose, 2% agar, pH 6.0) and incubated at 28°C for 2 weeks.

After termination of incubation, 50mL of sterilized water was added, and the mixture shaken, filtered through 4 layers of gauze, centrifuged at 8,000 x g for 10 minutes, and suspended in 50mM tris buffer solution (pH 7.5) to give a spore suspension. To the spore suspension of 1x10⁶/mL (1.5mL) was added 0.5mL of 0.5% NTG (N-methyl-N'-nitro-N-nitrosoguanidine) solution, and the mixture was allowed to stand at 28°C for 15 minutes for mutation. 10% Na₂S₂O₃ (3mL) was added, and the mixture was centrifuged at 5,500 x g for 10 minutes and washed with sterilized water to give an NTG-treated spore suspension.

The NTG-treated spore suspension was plated on a GY agar medium (1% glucose, 0.5% yeast extract, 0.005% Triton X-100, 1.5% agar, pH 6.0) and cultivated at 28°C, and the colony was transplanted to another GY agar medium. A part of the grown cells was dried, and extracted with hexane according to a conventional manner to obtain triglycerides; hexane was distilled off, and the resulting triglycerides were analyzed by high performance liquid chromatography. As a result of an examination of approximately 3,000 colonies, three mutants (#1 strain, #2 strain and #3 strain) were obtained in which the rate of triarachidonoyl glycerol contained in the total triglycerides was increased.

The rates (% by weight) of triarachidonoyl glycerol in the total triglycerides produced by #1, #2 and #3 strains were 17.0%, 15.1% and 12.2%, respectively, and in all other colonies the rates were 8% or lower. The rates (% by weight) of triarachidonoyl glycerol in the total triglycerides produced by #1, #2 and #3 strains were determined by means of a differential refractometer according to a conventional manner using a COSMOSIL 5C18-MA column (4.6 x 250mm) as an analytical column with acetonitrile/acetone (1:1) as a mobile phase (1.0mL/min).

### Example 2

### Comparison of the productivity of triarachidonoyl glycerol

Onto 10mL of liquid medium (2% glucose, 1% yeast extract, and 1% palmitoleic acid or no palmitoleic acid) contained in a 50mL Erlenmeyer flask was inoculated respectively a platinum loop of the following 3 strains of Mortierella alpina, and incubated at 28°C with shaking of 120rpm for 7 days.
(1) Mortierella alpina #1 (mutant obtained in Example 1)
(2) Mortierella alpina SAM 2268
(3) Mortierella alpina IFO 8568

After termination of incubation, the cells were collected by filtration. A part of the grown cells was dried, and extracted with hexane according to a conventional manner to obtain triglycerides, and hexane was distilled off to give triglycerides. The resulting triglycerides were applied to high performance liquid chromatography in order to analyze the molecular species and determine the rate of triarachidonoyl glycerol (tri-AA). In addition, a part of the grown cells was dried, and the fatty acids contained in the cells were subjected to methyl esterification with hydrochloric acid-methanol according to a conventional manner, and extracted with hexane; evaporation of hexane afforded fatty acid methyl esters. The resulting fatty acid methyl esters were analyzed by gas chromatography to determine the rate of arachidonic acid in the total fatty acids. Table 1 shows the results.

**Table 1. Productivity of triarachidonoyl glycerol (tri-AA)**

| Strain | #1 | SAM2268 | IFO856 |
|---|---|---|---|
| Rate of tri-AA in total triglycerides (% by weight) | 33.3 | 10.2 | 18.5 |
| Rate of arachidonic acid in total fatty acids (% by weight) | 48.2 | 38.8 | 45.6 |

The rate of tri-AA in the parent strain SAM 2268 was 10.2%, but the rate of tri-AA in the #1 mutant reached 33.3%. The rate of tri-AA in IFO of which the rate of arachidonic acid in the total fatty acids was the same level was only 18.5%.

### Example 3

### Aged deterioration of triarachidonoyl glycerol production

Onto 10mL of liquid medium (2% glucose, 1% yeast extract, and 1% palmitoleic acid or no palmitoleic acid) contained in a 50mL Erlenmeyer flask was inoculated a platinum loop of Mortierella alpina #1, and incubated at 28°C with shaking at 120rpm for 6, 8 or 10 days, respectively. After termination of incubation, the cells were collected by filtration, and the rates of triarachidonoyl glycerol (tri-AA) and of arachidonic acid in the total fatty acids were determined in the same manner as in Example 2. Table 2 shows the results.

**Table 2. Aged deterioration of triarachidonoyl glycerol (tri-AA) production in #1 strain**

| Number of days for culture | 6 | 8 | 10 |
|---|---|---|---|
| Rate of tri-AA in total triglycerides (% by weight), | 30.1 | 45.9 | 60.4 |
| Rate of arachidonic acid in total fatty acids (% by weight) | 45.6 | 53.2 | 62.2 |

The rate of tri-AA was 30.1% at the 6th day, but it reached 45.9% at the 8th day and 62.5% at the 10th day.

### Example 4

### Mass production of triarachidonoyl glycerol

A platinum loop of Mortierella alpina #1 was inoculated on 100mL of seed medium A and preliminarily incubated in a condition at 28°C with shaking of 100rpm for 3 days. Then, 5L of main culture medium D was placed in a 10L volume aeration fermenter equipped with a stirrer and sterilized. All of the above preliminarily incubated broth was inoculated thereon and incubated at 26°C with stirring of 300rpm under aeration of 1 vvm for 10 days. In accordance with consumption of glucose, approximately 1% glucose was properly fed. The resulting cultured cells were sterilized, and dried after removal of the culture medium to give 75g of dry cells.

To 75g of dry cells was added 225mL of hexane, and the mixture was gently stirred at a usual temperature for 3 hours and filtered to give a hexane layer. In addition, 150mL of hexane was added to the dry cells, then gently stirred at usual temperature for 3 hours and filtered to give a hexane layer. The hexane layers were combined and evaporated to give 35 g of crude oily extract. This was treated with active clay to give 33 g of triglycerides. A part of the triglycerides was analyzed to determine the rate of triarachidonoyl glycerol (tri-AA) and the rate of arachidonic acid in the total fatty acids in the same manner as in Example 2, indicating that the rates were 55.0% and 59.7%, respectively. A part of the triglycerides was applied to high performance liquid chromatography shown in Example 1 to separate a tri-AA fraction. Thus, 10 g of tri-AA was obtained.

### Example 5

### Lipase reaction using triarachidonoyl glycerol

Into 8ml of 12.5% Rhizopus delemar lipase aqueous solution (Talipase powder; Tanabe Seiyaku Co., Ltd.) was suspended 10g of ion-exchange resin carrier (Dowex MARATHON WBA; Dow Chemical; trade mark), and the mixture was dried under reduced pressure to give immobilized lipase.

Subsequently, 8g of caprylic acid triglyceride (MCT), 600mg of the above immobilized lipase and 240µl of water were allowed to react at 30°C with stirring (130rpm) for 48 hours. After the reaction completion, the reaction solution was removed to give an activated immobilized enzyme.

One g of triarachidonoyl glycerol (tri-AA) prepared in Example 4, 2g of caprylic acid, and 150mg of the above immobilized enzyme (Rhizopus delemar lipase; carrier: Dowex MARATHON WBA) were allowed to react at 30°C with stirring (130rpm) for 48 hours. In the fats or oils of the reaction mixture from which was removed the immobilized enzyme, there were arachidonic acid cleaved from tri-AA at 1,3-positions and excess of reaction substrate, caprylic acid; these fatty acids were removed by extraction with an alkali to give once-treated fats or oils. The resulting once-treated fats or oils (1g), 2g of caprylic acid, and 150mg of recovered immobilized enzyme were allowed to react at 30°C with stirring (130rpm) for 48 hours. The mixture was treated in the same manner as above to remove caprylic acid and so on and afforded 0.8g of twice-treated fats or oils. The latter contained 96 mole % 1,3-capryloyl-2-arachidonoyl glycerol.

### Example 6

### Preparation of capsules containing triarachidonoyl glycerol

Gelatin (Nitta Gelatin Co.) and glycerin for food additive (Kao Corporation) were mixed so as to be 100:35 by weight, to which water was added. The mixture was dissolved at a temperature of 50-60°C to give gelatin film of 2000cp in viscosity. Then, the triglycerides containing 55.0% of triarachidonoyl glycerol (tri-AA) prepared in Example 4 were mixed with vitamin E oil (Eisai Co.) in a ratio of 100:0.05 by weight to prepare a content. Using these materials, forming of capsules and drying were carried out in a conventional manner to give soft capsules containing 180mg of the content per capsule. These soft capsules were suitable for oral application.

### Example 7

### Preparation of beverage containing triarachidonoyl glycerol

Triglycerides containing 55.0% of triarachidonoyl glycerol (tri-AA) prepared in Example 4 were mixed with soybean lecithin (Tsuji Seiyu) in a ratio of 9:1 by weight, and dispersed homogeneously into water to give a liposome dispersion liquid. This liposome dispersion liquid was added to orange juice, carbonated water, coffee drink, milk, soymilk, or thick soup at a ratio of 1/100 by volume to give the corresponding drink as food concerning the invention. These drinks were very suitable for oral application.

The invention is not limited to the above-explained respective embodiment and may be modified in various ways within the scope of Claims. In addition, technical means disclosed respectively in different working modes and examples may be combined properly and the resulting working mode also falls in the technical scope of the invention.

### Industrial Applicability

As mentioned above, triglycerides constituted by 3 PUFA residues of one type can be produced efficiently and stably by the fermentation technique of the invention. The invention, accordingly, can be widely used particularly in an industrial field of functional foods as well as in a field of general foods and medicines.

## Claims

1. A process for producing fats or oils in which the rate of triglycerides constituted by the same polyunsaturated fatty acids linking to the three hydroxyl groups of glycerol is 20% or more by weight for the total triglycerides, which comprises culturing a microorganism capable of producing said fats or oils and, if required, recovering said fats or oils.

2. A process as claimed in Claim 1, wherein the rate of triglycerides constituted by the same polyunsaturated fatty acids linking to the three hydroxyl groups of glycerol is 30% or more by weight for the total triglycerides.

3. A process as claimed in Claim 2, wherein the rate of triglycerides constituted by the same polyunsaturated fatty acids linking to the three hydroxyl groups of glycerol is 40% or more by weight for the total triglycerides.

4. A process as claimed in Claim 3, wherein the rate of triglycerides constituted by the same polyunsaturated fatty acids linking to the three hydroxyl groups of glycerol is 50% or more by weight for the total triglycerides.

5. A process as claimed in any one of Claims 1 to 4, wherein the above-mentioned same polyunsaturated fatty acid is arachidonic acid.

6. A process as claimed in any one of Claims 1 to 5, wherein the above-mentioned microorganism is one of a genus Mortierella.

7. A process as claimed in Claim 6, wherein the above-mentioned microorganism is one of a subgenus Mortierella of a genus Mortierella.

8. A process as claimed in Claim 7, wherein the above-mentioned microorganism is one of a species Mortierella alpina.

9. A process as claimed in any one of Claims 1 to 8, wherein the above-mentioned microorganism is a mutant derived from a microorganism capable of producing fats or oils containing polyunsaturated fatty acids.

10. Fats or oils produced by the method as claimed in Claims 1 to 9.

11. A microorganism belonging to a genus Mortierella which is able to produce fats or oils in which the rate of triglycerides constituted by the same polyunsaturated fatty acids linking to the three hydroxyl groups of glycerol is 20% or more by weight for the total triglycerides.

12. A microorganism belonging to a genus Mortierella which is able to produce fats or oils in which the rate of triglycerides constituted by the same polyunsaturated fatty acids linking to the three hydroxyl groups of glycerol is 30% or more by weight for the total triglycerides.

13. A microorganism claimed in Claim 11 or 12, being Mortierella alpina.

14. A microorganism claimed in any one of Claims 11 to 13, being a mutant.

15. Fats or oils in which the rate of triglycerides constituted by the same polyunsaturated fatty acids linking to the three hydroxyl groups of glycerol is 20% or more by weight for the total triglycerides.

16. Fats or oils in which the rate of triglycerides constituted by the same polyunsaturated fatty acids linking to the three hydroxyl groups of glycerol is 30% or more by weight for the total triglycerides.

17. Fats or oils in which the rate of triglycerides constituted by the same polyunsaturated fatty acids linking to the three hydroxyl groups of glycerol is 40% or more by weight for the total triglycerides.

18. Fats or oils in which the rate of triglycerides constituted by the same polyunsaturated fatty acids linking to the three hydroxyl groups of glycerol is 50% or more by weight for the total triglycerides.

19. A cultured cell of a microorganism comprising fats or oils in which the rate of triglycerides constituted by the same polyunsaturated fatty acids linking to the three hydroxyl groups of glycerol is 20% or more by weight for the total triglycerides.

20. A cultured cell of a microorganism comprising fats or oils in which the rate of triglycerides constituted by the same polyunsaturated fatty acids linking to the three hydroxyl groups of glycerol is 30% or more by weight for the total triglycerides.

21. A cultured cell of a microorganism comprising fats or oils in which the rate of triglycerides constituted by the same polyunsaturated fatty acids linking to the three hydroxyl groups of glycerol is 40% or more by weight for the total triglycerides.

22. A cultured cell of a microorganism comprising fats or oils in which the rate of triglycerides constituted by the same polyunsaturated fatty acids linking to the three hydroxyl groups of glycerol is 50% or more by weight for the total triglycerides.
